# EUROPEAN PATENT SPECIFICATION

(11) **EP 3 471 676 B1**
(45) Date of publication and mention of the grant of the patent: **19.08.2020**
(21) Application number: 17732177.5
(22) Date of filing: 15.06.2017
(51) Int. Cl.: A61F 13/00, B41M 5/124, B41M 5/136

(54) **PRESSURE INDICATING DEVICES**
DRUCKANZEIGEVORRICHTUNGEN
DISPOSITIFS D'INDICATION DE PRESSION

(30) Priority: 20.06.2016 GB 201610712; 09.02.2017 GB 201702121
(43) Date of publication of application: 24.04.2019
(73) Proprietor: Active Device Development Limited, Stockton-on-Forest, York YO32 9LE (GB)
(72) Inventor: GREENER, Bryan, York Yorkshire YO10 4LZ (GB)
(74) Representative: Secerna LLP
(86) International application number: PCT/GB2017/051745
(87) International publication number: WO 2017/220972

(56) References cited:
- US-A1- 2010 326 198
- US-A1- 2013 033 755
- US-A1- 2015 283 839

## Description

### Field of Invention

Certain embodiments of the present invention concern pressure indicating sheet materials. In particular, but not exclusively certain embodiments concern reversible pressure indicating sheet materials that appear to exhibit a colour change, to the human eye or to an electronic means of detection, when a given pressure is applied to or removed from the device.

### Background to invention

Prior art is this area includes a children's toy called "Magic Slate" that is well described by US1455579, US2663095 and US8075982. Briefly, the magic slate comprises a milky or semi-opaque or translucent cover sheet overlying a waxed surface of dark colour. In use, a stylus or suchlike is used to apply pressure to the cover sheet, sufficient to embed it locally in the waxed surface. The intimate contact of the translucent sheet and waxed surface in this area result in locally increased light transmission, enabling the viewer to clearly distinguish the dark colour of the waxed surface. To re-set the apparatus, the cover sheet can be lifted from the waxed surface, breaking contact and removing any previously scribed marks. The wax remains permanently deformed by this process but can be re-smoothed by various inventions of the prior art (see US3381299 and US2003203349) that do not concern the present invention disclosed herein.

Key physical characteristics of the Magic Slate system are a semi-opaque or translucent cover sheet and a permanently deformable (i.e. mechanically plastic) wax surface. In this prior art, an applied pressure is required to create a semi-permanent bond between the two layers. This bond can only be broken by application of a separating force which, according to the prior art, may be generated by at least mechanical or pneumatic (see US3943643) means. This is a re-settable system (it does not reverse without a further stimulus).

An apparatus of the prior art that is distinct from the Magic Slate system is that described in US7568916B1 and involves the temporal displacement of a viscous liquid of high viscosity by an applied pressure. A backing of contrasting colour to the viscous liquid is revealed by the displacement. After a time, the viscous liquid returns to its original location and the image is erased. This is a reversible system.

### Summary of the Invention

According to a first aspect of the present invention, there is provided a device for reversible indication of applied pressure by a colour change comprising;
a translucent layer;
a coloured layer comprising an elastomer; and
an apertured layer, comprising one or more apertures;
wherein the translucent layer and the coloured layer are associated by means of the apertured layer.

Suitably, the translucent layer and the coloured layer are connected via the apertured layer.

Suitably, the apertured layer is interposed between the translucent layer and the coloured layer.

Suitably, the translucent layer comprises a first surface and second surface opposed to the first surface.

In a further aspect of the present invention, there is provided a device for the reversible indication of applied pressure by a colour change comprising a translucent layer and a coloured layer comprising an elastomer associated by means of an apertured layer, comprising one or more apertures.

As used herein, the term 'translucent' is taken to have the same meaning and be interchangeable with the terms milky, opalescent, semi-transparent and semi-opaque and all are taken to mean a material that does not transmit 100% of incident light from one face to the other but, instead, returns at least a portion of incident light as scattered and/or reflected light to the surface from which it was incident to. In certain embodiments of the present invention translucency of the translucent layer is provided by a texture on at least one surface of the translucent layer.

Suitably, the translucent layer is able to return 50-100% of incident light to the face from which it was incident.

More suitably, the translucent layer is able to return 50-70% of incident light to the face from which it was incident.

Suitably, the translucent layer is configured to reflect at least a portion of light incident to a face of the translucent layer by total internal reflection. As used herein the term "total internal reflection" refers to the phenomenon which occurs when a propagated wave such as light is incident to an interface between a first and second medium at an angle larger than a particular critical angle with respect to the normal to a surface of the first medium. If the refractive index of the first medium (the medium the light is initially incident to) is lower than the refractive index of the second medium and the incident angle is greater than the critical angle of the interface between the first and second medium, the light cannot pass through the first and second medium and is entirely reflected back to the surface the light was initially incident to. As used herein the term "the critical angle" refers to an angle of incidence above which total internal reflection occurs.

Suitably, at least one surface of the translucent layer is textured. Suitably, the texture can be regular or irregular.

Suitably, the regular or irregular surface of the textured translucent layer comprises features that are of a size in a range, in any dimension (such as for example height, width, and/or diameter), of 100 nanometres to 1 millimetre. Aptly the features are structural features. Aptly, structural features on this scale are capable of scattering and/or reflecting incident light, thus providing the material with translucency.

Suitably, when the surface of the textured translucent layer is irregular, it may comprise a void-rich structure, not limited to but including, a continuous layer comprising air-filled voids. Suitably, the air-filled voids are of a size, in any dimension, in a range of 100 nanometres to 1 millimetre.

Examples of materials with irregular surface texture with air-filled voids include tissue paper, paper, non-wovens, wovens, knits, open-celled foams and opacified polymer sheets. Opacified polymer sheets include those made of PVC (polyvinylchloride), PET (polyethyleneterephthalate) and cellulose acetate. Mylar A (3M) is a specific example of an opacified PET.

Suitably, the translucent layer comprises one or more projections extending from at least a portion of at least one surface of the translucent layer. Suitably the one or more projections have any shape, such as for example cuboid, triangular, spherical, parabolic, pyramidal, ellipsoidal, trapezoidal, prismatic or hemispherical. Aptly the one or more projections comprise a hemispherical shape. Aptly the projections comprise air filled voids.

Suitably, the one or more projections extend from the first face of the translucent layer.

Suitably, when the structure of the textured translucent layer is regular, it may comprise a lenticular lens array (also known as a microlens array).

As used herein the term "lenticular lens array" refers to a sheet or film having an array of lenticules, or micro-lenses, on at least one side of the sheet that focus light onto a focal plane and/or reflect light. Generally, the opposite side of the sheet is substantially flat.

Suitably, the lenticular lens array comprises a close packed arrangement of lenticules, arranged in a tetragonal or hexagonal close-packed manner.

Suitably, the lenticules may be hemispherical convex projections extending from at least a portion of at least one surface of the translucent layer. Aptly the lenticules extend from the first surface of the translucent layer.

Suitably, the lenticules are of a size, in any dimension, in a range of 100 nanometres to 1 millimetre.

Suitably, the lenticular lens array is in the form of a sheet with the lenticules disposed on a face and an opposing face is flat.

Suitably, the sheet comprises a first surface and a second surface opposed to the first surface wherein the lenticular lenses are disposed on the first surface and the second surface is flat.

Suitably, the lenticular lens array is constructed of a transparent material.

Aptly the translucent layer comprising the lenticular lens array is constructed from a transparent material and the structure of the lenticular lenses are configured to scatter or reflect light incident to a surface of the translucent layer and return this light to the surface the light was incident to and thus provide the transparent material with translucency. Aptly the light is incident to the second surface of the translucent sheet.

As used herein the term "transparent" refers to non-opaque or at least less opaque materials than translucent materials. Transparency can be measured in terms of percentage and/or intensity of light that passes or transmits through the material using, for example, a photospectrometer.

Suitably, the lenticular lens array is constructed of a polymeric material such as, but not limited to, polypropylene, poly(methyl methacrylate), polycarbonate or poly(ethylene terephthalate) or a combination of these.

Suitably, the coloured layer comprising an elastomer is a coloured elastomer. Suitably, the coloured elastomer can be opaque or transparent.

Suitably, the elastomer has a Shore hardness on the Shore OOO or Shore OO or Shore A scales.

Suitably, the coloured layer comprising an elastomer is a transparent, uncoloured elastomer directly associated with a coloured backing sheet.

Aptly the coloured layer comprises a transparent elastomer. Aptly the elastomer is uncoloured. Aptly, the coloured layer comprises a coloured backing sheet. Aptly the transparent elastomer is in direct contact with at least a portion of the coloured backing sheet.

Suitably, the elastomer has a Shore hardness on the Shore OOO or Shore OO or Shore A scales.

Suitably, the elastomer is selected from a hydrocarbon-based gel, a polyurethane gel, a hydrogel and a silicone based gel.

Suitably, each of the elastomer and the translucent layer have a refractive index in a range of 1.30-1.71. Suitably, each of the elastomer and the translucent layer have a substantially similar refractive index.

Aptly, the elastomer has a refractive index in a range 1.40-1.60. For example, the elastomer may have a refractive index of 1.40, aptly 1.41, aptly 1.42, aptly 1.43, aptly 1.44, aptly 1.45, aptly 1.46, aptly 1.47, aptly 1.48, aptly 1.49, aptly 1.50, aptly 1.51, aptly 1.52, aptly 1.53, aptly 1.54, aptly 1.55, aptly 1.56, aptly 1.57, aptly 1.58, aptly 1.59, aptly 1.60. Suitably, the elastomer is a silicone elastomer. Suitably, the elastomer is a gelled hydrocarbon elastomer.

Aptly, the translucent layer has a refractive index in a range of 1.30-1.71. For example, the translucent layer may have a refractive index of 1.30, aptly 1.31, aptly 1.32, aptly 1.33, aptly 1.34, aptly 1.35, aptly 1.36, aptly 1.37, aptly 1.38, aptly 1.39, aptly 1.40, aptly 1.41, aptly 1.42, aptly 1.43, aptly 1.44, aptly 1.45, aptly 1.46, aptly 1.47 aptly, 1.48, aptly 1.49, aptly 1.50, aptly 1.51, aptly 1.52, aptly 1.53, aptly 1.54, aptly 1.55, aptly 1.56, aptly 1.57, aptly 1.58, aptly 1.59, aptly 1.60, aptly 1.61, aptly 1.62, aptly 1.63, aptly 1.64, aptly 1.65, aptly 1.66, aptly 1.67, aptly 1.68, aptly 1.69, aptly 1.70, aptly 1.71.

Suitably, the apertured layer comprising one or more apertures may comprise a single aperture or multiple substantially identical apertures.

Suitably, the apertured layer comprises a single aperture that is formed when the translucent layer and the coloured layer comprising an elastomer are associated by a spacer ring (e.g. a flat washer) that may take any geometry, such as an annulus (circular), square frame or ellipse. Suitably, the spacer ring (e.g. a flat washer) takes the shape of an annulus.

Suitably, the apertured layer comprises more than one aperture of regular geometry.

Suitably, the many apertured layer may be constructed of a woven, non-woven or knitted material or an otherwise apertured (e.g. perforated or slit and extended) continuous material. When the apertured layer is a woven or knitted material, it is suitably constructed of a monofilament yarn.

According to a third aspect of the present disclosure there is provided a clothing garment comprising a pressure indicator device as described herein.

According to a fourth aspect of the present invention, there is provided a negative pressure wound therapy device comprising a pressure indicator device as described herein.

According to a fifth aspect of the present invention, there is provided a compression garment comprising a pressure indicator device as described herein.

According to a sixth aspect of the present invention, there is provided a kit comprising a pressure indicator device for reversible indication of applied pressure by a colour change comprising;
a translucent layer;
a coloured layer comprising an elastomer; and
an apertured layer, comprising one or more apertures;
wherein the translucent layer and the coloured layer are connected by means of the apertured layer; and
a medical device.

In certain embodiments, the kit comprises a pressure indicator device as described herein.

In certain embodiments, the medical device comprises a negative pressure wound therapy device.

In certain embodiments, the medical device comprises a compression garment.

Suitably, the device is integral with the medical device.

Suitably, the device is separable to the medical device.

According to a seventh aspect of the present disclosure, there is provided use of a pressure indicator device as described herein or a kit as described herein for indicating pressure applied by a negative pressure wound therapy device.

According to an eighth aspect of the present disclosure, there is provided use of a pressure indicator device as described herein or a kit as described herein for indicating pressure applied by a compression garment.

According to a ninth aspect of the present invention, there is provided use of a pressure indicator device as described herein or a kit as described herein for indicating pressure applied by a clothing garment.

According to a tenth aspect of the present disclosure, there is provided a method of reversibly detecting pressure change comprising the steps of;
providing a pressure indicator device as described herein or a kit as described herein;
increasing or decreasing a pressure applied to the device; and
detecting a change in colour of at least a portion of the device subsequent to the increase or decrease in the pressure applied.

Suitably, detecting the change in colour comprises visually detecting the change in colour.

Suitably, the change in colour is detected using an electronic device.

Suitably, detecting the change in colour comprises detecting the change in colour using an electronic device.

### Brief Description of the Drawings

Certain embodiments of the present invention will now be described in more detail below, by way of example only, with reference to the accompanying drawings, in which:

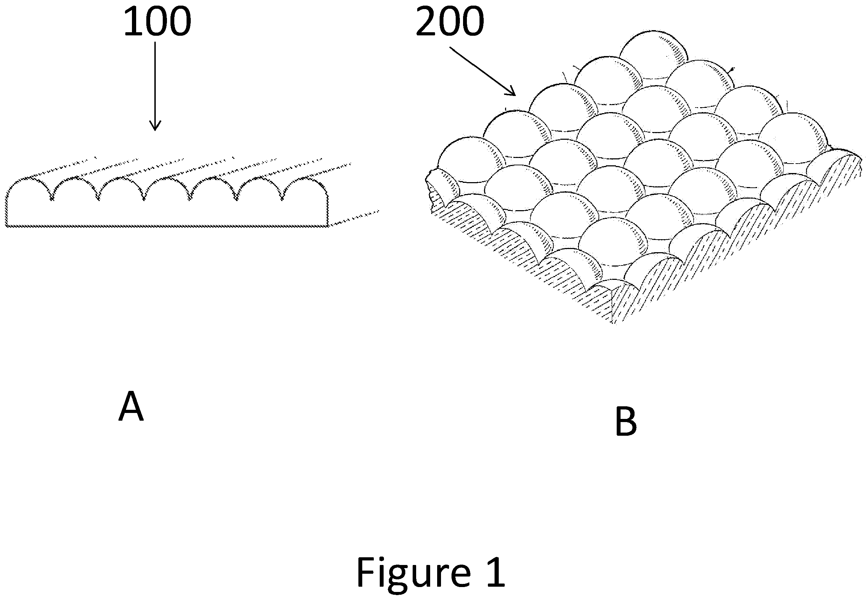

Figure 1A depicts a linear lenticular lens array (100) and Figure 1B depicts a tetragonal close-packed lenticular lens array (200).

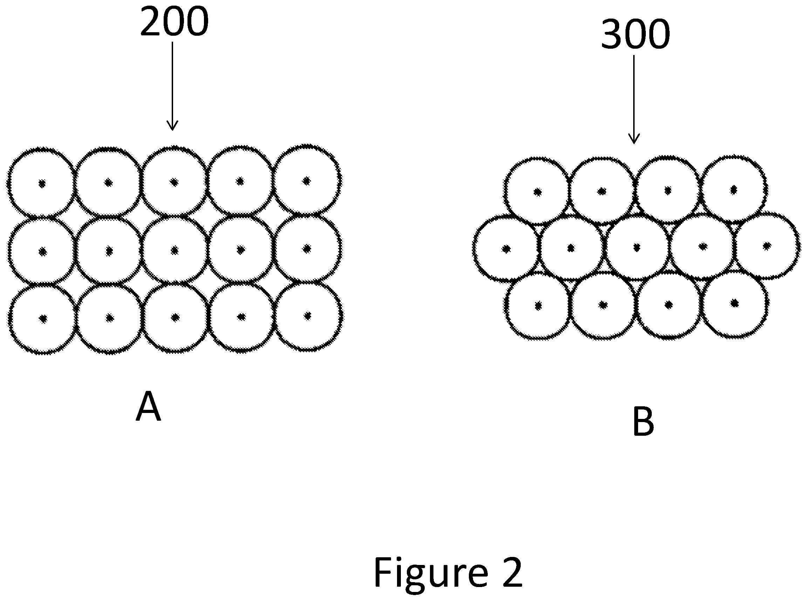

Figure 2A depicts, in plan view, a tetragonal close-packed lenticular lens array (200) and Figure 2B depicts a hexagonal close-packed lenticular lens array (300).

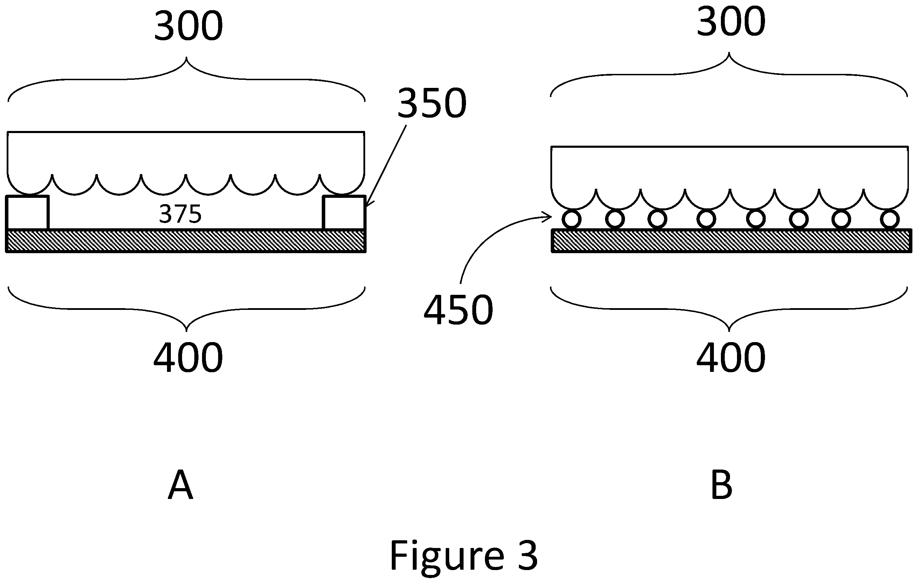

Figure 3A depicts, in cross-section, a tetragonal close packed lenticular lens array (300) and a coloured layer comprising an elastomer (400), associated by means of a single aperture (375) created by a spacing ring (350). Figure 3B depicts, in cross section, a tetragonal close packed lenticular lens array (300) and a coloured layer comprising an elastomer (400), associated by means of an apertured woven mesh (450).

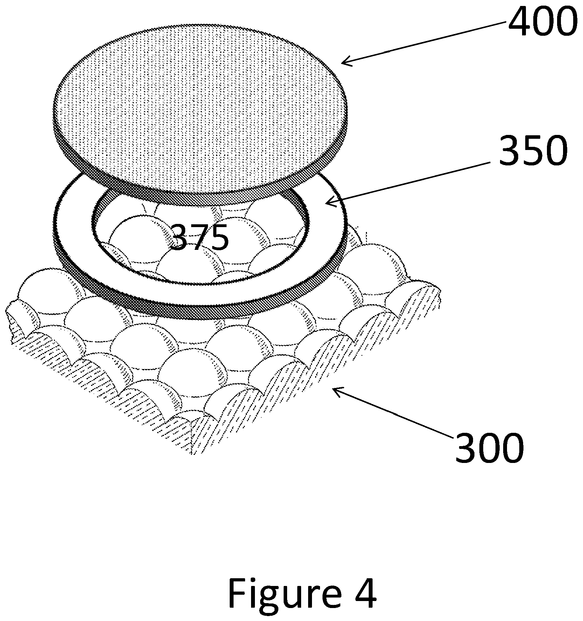

Figure 4 is an isometric view of Figure 3A, depicting a tetragonal close packed lenticular lens array (300) and a coloured layer comprising an elastomer (400), associated by means of a single aperture (375) created by a spacing ring (e.g. a flat washer) (350).

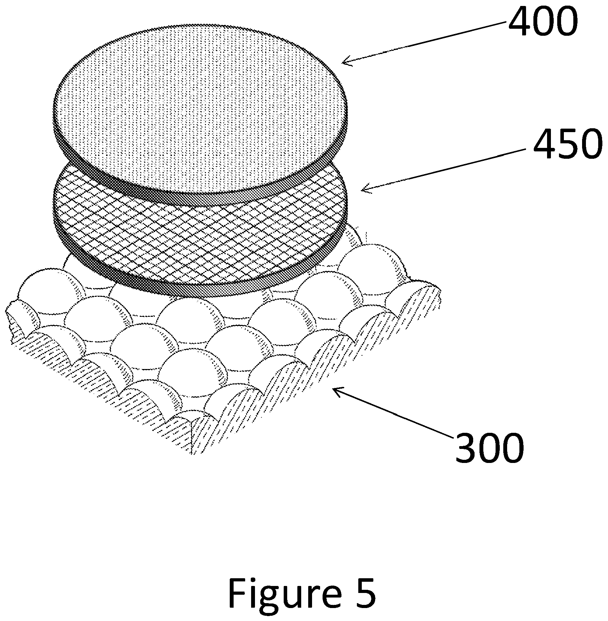

Figure 5 is an isometric view of Figure 3B, depicting a tetragonal close packed lenticular lens array (300) and a coloured layer comprising an elastomer (400), associated by means of an apertured woven mesh (450).

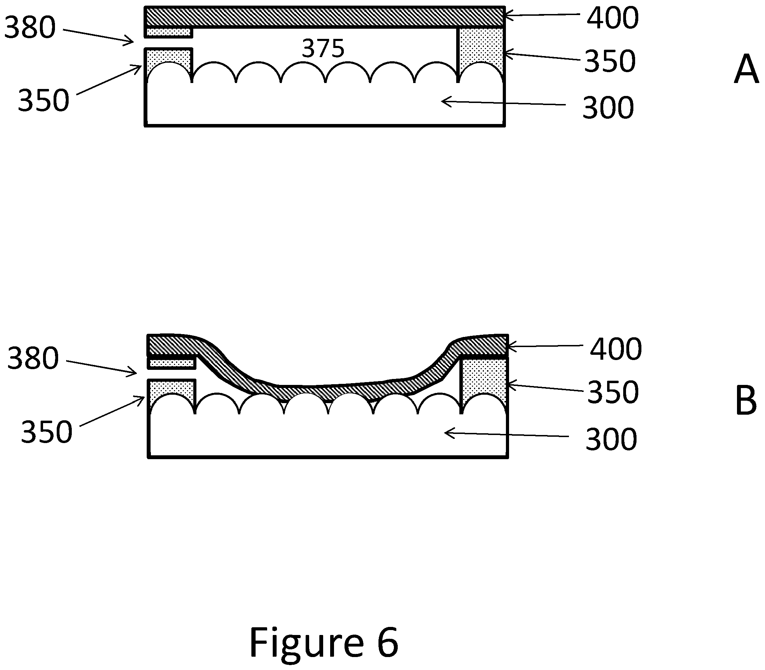

Figure 6A is a schematic cross-section view of an embodiment of the present invention suitable for the indication of a pressure below ambient pressure (i.e. a vacuum) comprising, a hexagonal close packed lenticular lens array (300) and a coloured layer comprising an elastomer (400), associated by means of a single aperture (375) created by a spacing ring (350), that is gas-tightly sealed to the lenticular lens array (300) and the elastomer (400), that contains an aperture in its wall (380) for attachment to a source of vacuum. Figure 6B is a schematic cross-section view of an embodiment of the present invention suitable for the indication of a pressure below ambient pressure (i.e. a vacuum), as shown in Figure 6A, and now connected to a vacuum source via aperture (380). Ambient pressure has exerted a pressure on the elastomer (400), deforming it into direct contact with the lens array (300).

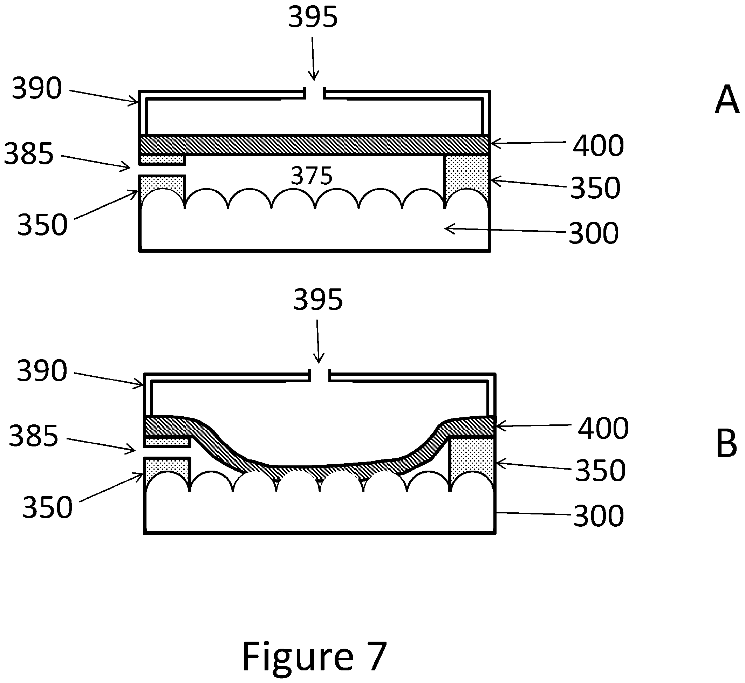

Figure 7A is a schematic cross-section view of an embodiment of the present invention suitable for the indication of a pressure above ambient pressure comprising, a hexagonal close packed lenticular lens array (300) and a coloured layer comprising an elastomer (400), associated by means of a single aperture (375) created by a spacing ring (350) that contains an aperture in its wall (385) to avoid air-locking. The coloured layer comprising an elastomer (400) is also associated, on the opposing face to the spacing ring (350), to a rigid cavity (390) that contains an aperture in its wall (395) for attachment to a source of pressure. Figure 7B is a schematic cross-section view of an embodiment of the present invention suitable for the indication of a pressure above ambient pressure, as shown in Figure 7A, and now connected to a source of pressure via aperture (395). The pressure has deformed the elastomer (400) into direct contact with the lenticular lens array (300).

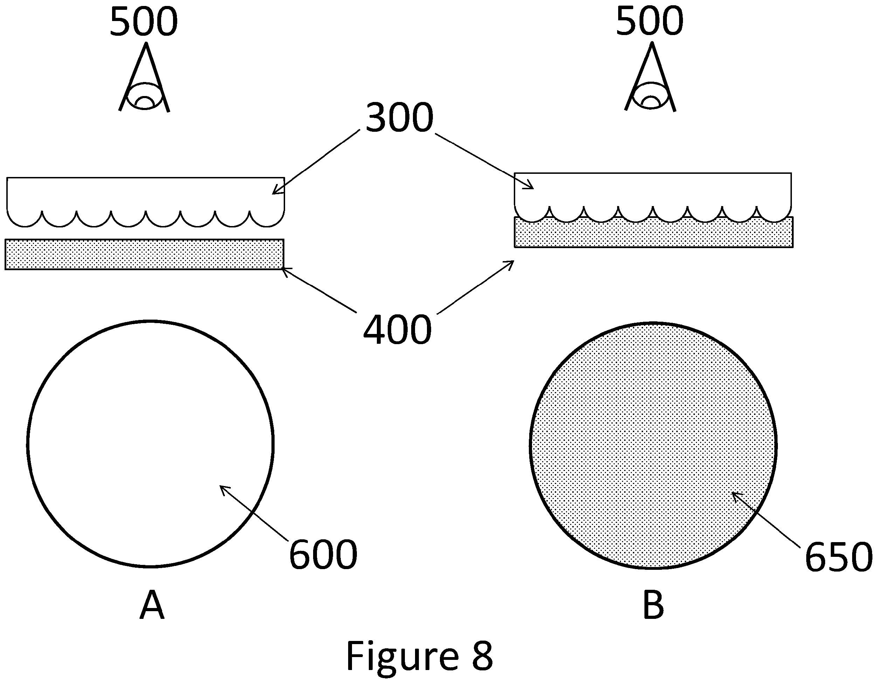

Figure 8A is a plan view and cross-section view of a hexagonal close packed lenticular lens array (300) and a coloured layer comprising an elastomer (400) in a non-contact state. Viewed (500) from the flat side of the lens array, the observer sees white light (600) reflected from the lenticular lens array by total internal reflection of incident light. Figure 8B is a plan view and cross-section view of a hexagonal close packed lenticular lens array (300) and a coloured layer comprising an elastomer (400) in a direct contact state. Viewed (500) from the flat side of the lens array, the observer sees the colour of the elastomer (650).

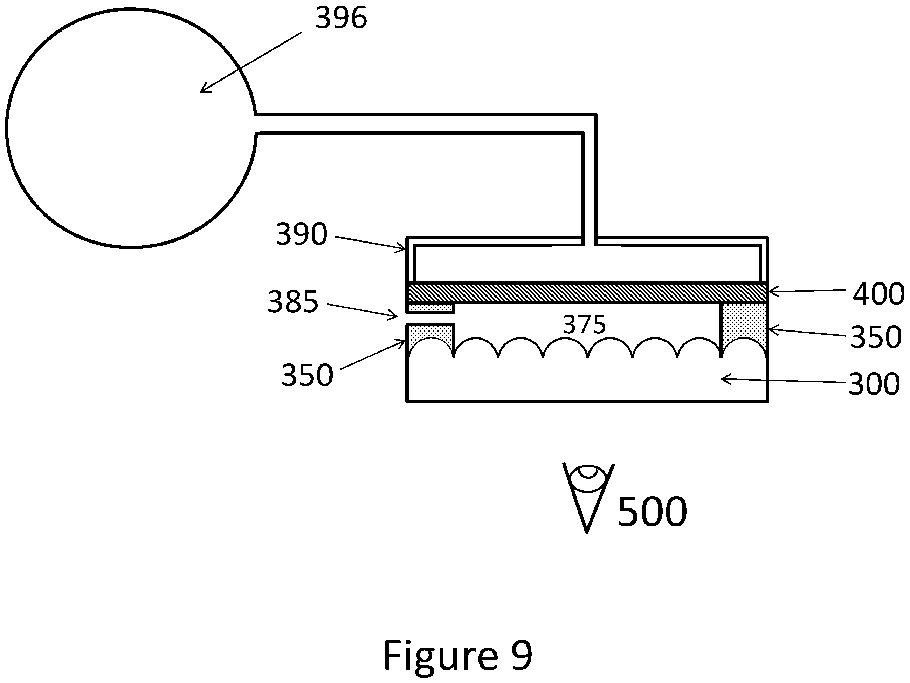

Figure 9 is a cross-section view of a pressure indicating device, as described and shown in Figure 7, connected to a flexible bladder (396). The flexible bladder can be compressed to generate a positive pressure inside the rigid cavity (390) to deform the coloured elastomer (400) and bring it into contact with the lenticular lens array (300). This contact can be visualised by a viewer (500), as described in Figure 8.

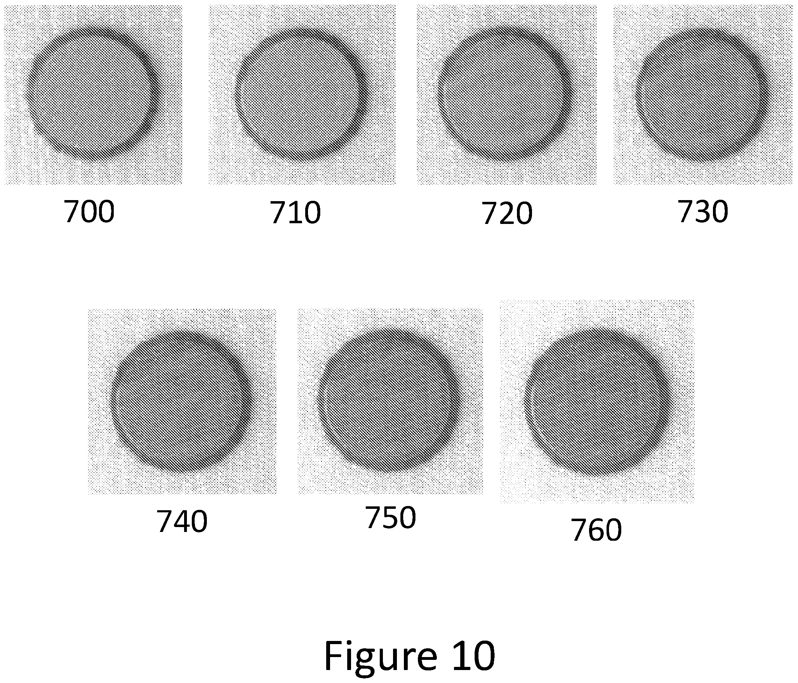

Figure 10 is a grey-scale (from blue) image of a pressure sensor constructed using a woven mesh multiple-apertured layer, as described in Examples 1 and 3 and shown in Figure 5. The sensor is exposed to a range of pressures on the inner wall of a vacuum enclosure: Atmospheric pressure (700), -20 mmHg (710), -40 mmHg (720), -60 mmHg (730), -80 mmHg (740), -100 mmHg (750) and -120 mmHg (760).

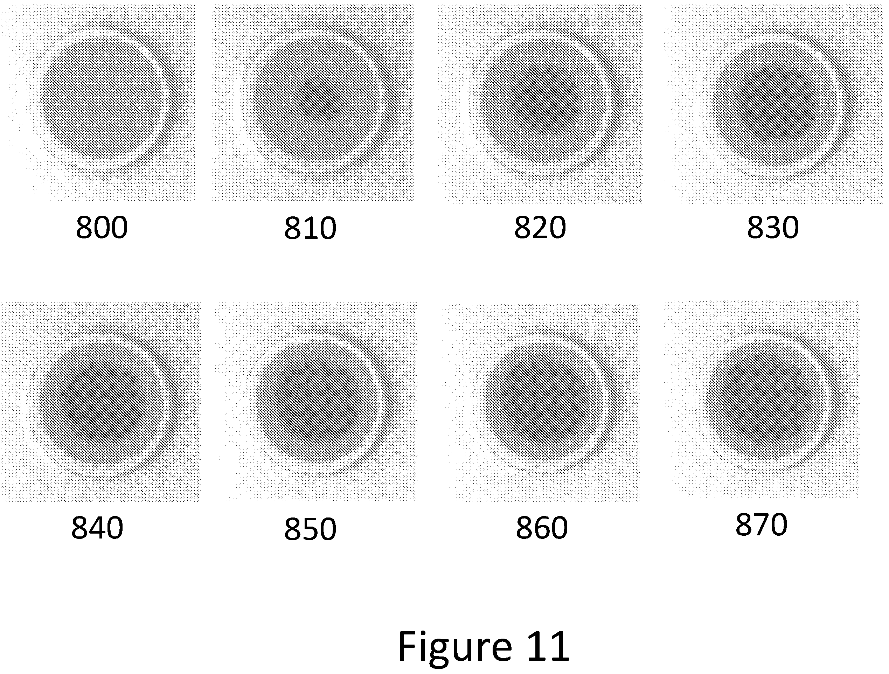

Figure 11 is a grey-scale (from blue) image of a pressure constructed using a single aperture, as described in Examples 2 and 4 and shown in Figure 4. The sensor is exposed to a range of pressures on the inner wall of a vacuum enclosure: Atmospheric pressure (800), -10 mmHg (810), -20 mmHg (820), -30 mmHg (830), -40 mmHg (840), -50 mmHg (850), -60 mmHg (860) and -70 mmHg (870).

### Description of Certain Embodiments of the Present Invention

According to a first aspect of the present invention there is provided a pressure indicator device for the reversible indication of applied pressure by a colour change comprising a translucent layer and a coloured layer comprising an elastomer associated by means of an apertured layer, comprising one or more apertures.

Herein, the term 'translucent' is taken to have the same meaning and be interchangeable with the terms milky, opalescent, semi-transparent and semi-opaque and all are taken to refer to a material that does not transmit 100% of incident light from one face to the other but, instead, returns at least a portion of incident light as scattered or reflected light to the surface from which the light was incident to.

Suitably, the translucent layer comprises a first surface and second surface opposed to the first face.

Suitably, the translucent layer is able to return 50-100% of incident light to the face from which it was incident.

More suitably, the translucent layer is able to return 50-70% of incident light to the face from which it was incident.

Suitably, the translucent layer is configured to return 50-100% of incident light to the face from which the light was incident to.

More suitably, the translucent layer is configured to return 50-70% of incident light to the face from which the light was incident to.

Aptly the incident light is incident to the second surface of the translucent layer.

Suitably, a surface of the translucent layer is textured. Suitably, the texture can be regular or irregular.

Suitably the first surface of the translucent layer is textured.

Suitably, the regular or irregular surface of the textured translucent layer comprises features that are of a size in any dimension (such as height, diameter, width and/or length) in a range of 100 nanometres to 1 millimetre. Aptly the features are one or more structural features. Without being bound by theory, structural features on this scale are capable of scattering and/or reflecting incident light.

Suitably, when the structure of the textured translucent layer is irregular, it may comprise a void-rich structure, not limited to but including, a continuous layer comprising air-filled voids.

Suitably, the air-filled voids are of a size, in any dimension, in a range of 100 nanometres to 1 millimetre.

Examples of materials with irregular surface texture with air-filled voids include tissue paper, paper, non-wovens, wovens, knits, open-celled foams and opacified polymer sheets. Opacified polymer sheets include those made of PVC (polyvinylchloride), PET (polyethyleneterephthalate) and cellulose acetate. Mylar A (3M) is a specific example of an opacified PET.

Suitably, the one or more structural features comprise one or more protrusions extending from at least one surface of the translucent layer. Aptly the structural features are hemispherical convex protrusions. Aptly the structural features are configured to scatter and/or reflect at least a portion of light that is incident to a surface of the translucent layer. Aptly the structural features extend from the first surface of the translucent layer.

Suitably, when the structure of the textured translucent layer is regular, it may comprise a lenticular lens array (also known as a microlens array).

Suitably, the lenticular lens array comprises a close packed arrangement of lenticules, arranged in a tetragonal or hexagonal close-packed manner.

Suitably, the lenticules are of a size, in any dimension, in a range of 100 nanometres to 1 millimetre.

Suitably, the lenticular lens array is in the form of a sheet with the lenses disposed on one face and an opposing face is flat.

Suitably, the lenticular lenses are disposed on the first surface of the translucent layer and the second opposing surface is flat.

Suitably, the face of the sheet with the lenses disposed on it is positioned proximal to the coloured layer comprising an elastomer (refer to Figure 3 and associated description).

Suitably, the lenticular lens array is constructed of a transparent material.

Suitably, the lenticular array is of a sheet thickness in a range of 10-5000 microns. More suitably, the lenticular array is of a sheet thickness in a range of 50-1000 microns.

Suitably, the lenticular array has a lens density in a range of 10-500 LPI (lens per linear inch). More suitably, the lenticular array has a lens density in the range of 50-500 LPI (lens per linear inch).

Suitably, each lens has a base diameter in a range of 10-1000 microns.

Without being bound by theory, light incident to the second substantially flat surface of the translucent layer is reflected and/or scattered by the lenticular lens array. Therefore, the intensity of light that returns to the second surface is increased, thus providing the translucent layer with translucency.

Without being bound by theory, low sheet thickness, high lens density, and small lens size may all contribute to a pressure imaging device with high spatial resolution.

The material from which the lens array is formed can be any substantially transparent, deformable polymeric material known to the skilled artisan including, but not limited to: nylons, acrylic resins, poly(ethylene terephthalate), poly(methyl methacrylate), polycarbonate, poly(vinylchloride), polystyrene, polyethylene or polypropylene or blends of these materials. These materials cover a broad spectrum of mechanical properties. Plasticising additives can be added to each plastic to control its hardness.

Suitable lenticular lens array sheet materials are available from OK3D Technology Co., Ltd, Film Optics Ltd and Forward Optics Incorporated for example.

Suitably, the coloured layer comprising an elastomer is a transparent, uncoloured elastomer directly associated with a coloured backing sheet.

In such embodiments, the colour of the coloured backing sheet is visible through the transparent, uncoloured elastomer.

Suitably, the backing sheet may be a coloured plastic sheet constructed of for example, but not limited to, a poly(carbonate), poly(ester) or cellulose-based film. Suitable materials are supplied by Rosco Laboratories Inc.

Suitably, the coloured layer comprising an elastomer is a coloured elastomer. Suitably, the coloured elastomer can be opaque or transparent.

Suitably, the elastomer has a Shore hardness on the Shore OOO, Shore OO or Shore A scales.

Suitably, the elastomer comprises a hydrocarbon-based gel, a polyurethane gel, a hydrogel or a silicone based gel.

Suitably, the elastomer comprises a hydrocarbon-based gel. Suitably, the hydrocarbon-based gel comprises a hydrocarbon and a co-polymer. Suitably, the hydrocarbon based gel comprises about 70% to about 98% by weight of a hydrocarbon, and up to about 30% by weight of a co-polymer selected from the group consisting of a triblock, radial block and multiblock copolymers, and optionally from 0 to about 10% by weight of a diblock copolymer. Apt co-polymers include, but are not limited to, styrene-ethylene/butylene-styrene (SEBS) copolymers and styrene-ethylene-propylene (SEP) copolymers. These co-polymers are supplied commercially by Kraton Performance Polymers Inc. under the Kraton G SEBS and SEPS trade names, and Versalis S.p.A. under the Europrene trade name. Suitable pre-formulated hydrocarbon-based gels include for example those described in US Patent No. 6066329 (Pennzoil Products Company). These materials are owned and marketed by Calumet Penreco Inc. under the trade name Versagel. These materials are solids at 25 °C and pourable liquids at temperatures above 80 °C. Materials of the Versagel C and Versagel R product ranges are all suitable for use as the elastomer of certain embodiments of the present invention.

The elastomers described herein comprise an organic liquid, for example but not limited to: mineral oil, isohexadecane, isododecane, hydrogenated polyisobutene, C12-C15 alkyl benzoate, isononyl isononanoate, isopropyl palmitate, petrolatum, squalane and hydrogenated poly(C6-C12 olefin).

Suitably, when the coloured layer comprising an elastomer is a coloured elastomer, the elastomer can be coloured by the addition of any suitable dye or pigment.

Suitably, the elastomer is coloured with a dye. Suitably the dye dissolves fully in the elastomer. Suitably, for colouring hydrocarbon-based elastomers, the dye is a solvent dye. Herein the term "solvent dye" is taken to mean a dye that is soluble in organic liquids.

Suitable solvent dyes include, but are not limited to, Solvent Black 3, Solvent Blue 35, Solvent Green 5, Solvent Red 23 and Solvent Yellow 124. These solvent dyes are available from FastColours LLP.

Suitably the elastomer comprises a silicone-based polymer. Suitable silicone-based polymers include the Silastic range of silicone elastomers (Dow Corning Corp.) and the Elastosil (Silpuran) range of silicone elastomers (Wacker Chemie AG).

Suitably, for colouring silicone-based elastomers, the dye is a silicone-compatible dye.

Suitable silicone-based dye formulations include, but are not limited to, the Elastosil AUX Color Paste FL range of Wacker Chemie AG.

Suitably, the apertured layer comprising one or more apertures may comprise a single aperture or multiple substantially identical apertures.

Suitably, the apertured layer has a thickness in a range of 0.01 mm to 5 mm. More suitably, the apertured layer has a thickness in the range of 0.1 mm to 2 mm.

Suitably, the apertured layer comprises a single aperture that is formed when the translucent layer and the coloured layer comprising an elastomer are associated by a flat washer that may take any geometry, such as an annulus (circular), square frame or ellipse. Suitably, the flat washer takes the shape of an annulus.

Suitably, single apertured layers comprise sheet materials formed in the shape of an annulus (e.g. a flat washer) or materials formed in the shape of a torus (e.g. circular grommets). Suitably, flat washer-type spacers are formed from sheet materials such as paper, cardboard, polypropylene, polyethylene, poly(tetrafluoroethylene), poly(carbonate), poly(vinylchloride) or poly(ethyleneterephthalate).

Suitably, single apertured layers have an aperture of regular geometry. Suitably, the single aperture is circular in shape and has a diameter in a range of 0.1-50 mm.

Suitably, the apertured layer comprises more than one aperture of regular geometry.

Suitably, the many apertured layer may be constructed of a woven, non-woven or knitted material or an otherwise apertured (e.g. perforated or slit and extended) continuous material. When the apertured layer is a woven or knitted material, it is suitably constructed of a monofilament yarn.

Non-limiting examples of apertured materials include the woven and knitted materials of Harrington Fabric and Lace (Nottingham, England) including item numbers: 4924, 4926 and 4917 and the bobbinet materials of Swisstulle UK Ltd (Nottingham, England) including article numbers: 182/28, 186, 188 and 198.

Suitably, multiple apertured layers have apertures of regular geometry. Suitably, the apertures are square, tetragonal or hexagonal in shape and have a major axis length in a range of 0.01-5 mm.

Certain embodiments of the present invention are designed for the indication of pressures below ambient pressure (i.e. vacuum). Certain embodiments of this kind require the coloured elastomer to be brought into contact with the translucent sheet (e.g. translucent layer comprising the at least one textured surface (e.g. the lenticular lens array)) when under an applied vacuum. This can be achieved in any way know to the skilled artisan, including the following: a vacuum source can be connected directly to the sealed aperture associating the coloured elastomer and translucent sheet (see Figure 6 where the translucent sheet is a lenticular lens array); or the device can be placed inside a flexible chamber to which a vacuum source can be connected. Ambient pressure causes the flexible chamber to change shape (e.g. a portion of the flexible chamber collapses) and exert a positive pressure on the contained device, bringing the coloured elastomer into contact with the translucent sheet.

Certain embodiments of the present invention are designed for the indication of pressures above ambient pressure. These embodiments require the coloured elastomer to be brought into contact with the translucent sheet under an applied pressure. This can be achieved in any way know to the skilled artisan, including the following: a sealed chamber can be associated with a face of the coloured elastomer distal to the translucent sheet, the sealed chamber has an aperture that can be connected to the source of pressure, see Figure 7.

The pressure at which the elastomer can contact the translucent sheet is determined by: the mechanical properties of the translucent sheet and the elastomer and the geometry of the apertured spacer. Commercially available translucent sheets are produced in a limited range of materials that are relatively inflexible; therefore pressure response variations are created herein by adjusting the material properties of the elastomer and the geometry of the apertured spacer. The current inventor has shown that pressures as low as 1-10 mmHg can be indicated when using a single aperture of appropriate geometry. The current inventor has also shown that pressures as low as 10-100 mmHg can be spatially imaged by a multi-apertured layer. Pressures as high 200000 mmHg can be indicated and imaged by some embodiments of the present invention, utilising relatively stiff elastomers and relatively small apertures of the apertured layer.

Certain embodiments of the pressure indicating devices described herein may have utility in the indication and/or imaging of pressure. The pressure may be greater than atmospheric pressure (a positive pressure) or may be lower than atmospheric pressure (a negative pressure or vacuum).

Without being bound by theory, the textured surface of the translucent layer (e.g. the lenticular lens array) reflects at least a portion of light when viewed from its flat face (the second opposing surface) and can appear translucent (e.g. opaque or semi-opaque) in light. Reflection of at least a portion of light incident to the opposing face may occur by the process of total internal reflection and relies upon the light reaching an interface between one medium (i.e. the translucent layer) and a less dense medium (e.g. air located within the aperture(s) of the apertured layer) when the angle of incidence of the incident light exceeds the critical angle of the interface between the two mediums. For example, a poly(propylene) and a poly(ethylene terephthalate) lens arrays exemplified here, have critical angles with air of 42 degrees and 38 degrees respectively. The approximately hemispherical lenticular lens shape ensures that a high percentage of light incident to the opposing flat side of the lenticular lens array is reflected back out of the same opposing flat face via total internal reflection by the lenticules. Thus, when the elastomer is not in contact with the lens array, its colour or the colour of the backing sheet is at least partially obscured to the viewer (see Figure 8A). Application of a pressure (positive or negative) can bring at least a portion of the elastomer into direct contact with at least a portion of the textured surface (lenticular lens array). The refractive index of the elastomer is sufficiently similar to that of the lens array to effectively eliminate total internal reflection due to what is referred to in the art as index matching. As used herein the term "refractive index matching materials" and "index matching materials" refer to two materials or substances that have approximately similar or substantially the same refractive indices as each other (e.g. the translucent layer and the elastomer). When index matching materials are in relatively close proximity of each other (e.g. in direct contact) incident light may pass from one material to the other with little to no reflection and/or refraction. Thus, the colour of at least a portion of the coloured layer becomes visible or more visible to the viewer when the elastomer is in direct contact with the textured surface of the translucent layer (see Figure 8B). When the applied pressure is removed, the elastomer separates from the textured surface and the colour of the coloured layer is less visible or is obscured to the viewer (see Figure 8A).

Aptly, the elastomer has a refractive index which is more similar to the refractive index of the translucent film than air and thus less likely to generate total internal reflection across the range of possible incident angles.

Aptly, the elastomer has a refractive index in the range 1.40-1.60. Examples of suitable elastomers include but are not limited to, silicone based gel elastomers, hydrocarbon-based gel elastomers, a polyurethane gel elastomers and hydrogel elastomers.

Examples of suitable translucent layer materials include but are not limited, poly(ethylene terephthalate) (refractive index: 1.636), poly(propylene) (refractive index: 1.49), poly(vinylchloride) (refractive index: 1.60), cellulose (refractive index: 1.54), poly(vinyl acetate) (refractive index: 1.466), poly(methyl methacrylate) (refractive index: 1.49), poly(carbonate) (refractive index: 1.58).

The intensity of the visible colour of the coloured layer and/or intensity of the returned incident light through and/or from at least a portion of the translucent layer can be detected by visual detection (e.g. by a user's eye or by an electronic detection device such as photospectrometer). Aptly, a dimension (such as diameter, width, length and/or area) of the portion of coloured layer visible upon application of a pressure may be detected.

Certain embodiments of the present invention may allow for multiple pressure application and removal of pressure cycles to be detected (i.e. the indication of pressure is reversible and repeatable). Certain embodiments may also provide a quantifiable indication of the amount of pressure applied, as greater pressure application provides an incremental increase or decrease in the intensity of the colour (of the coloured layer) visible through the translucent layer and/or dimension of the portion of the visible coloured layer.

Certain embodiments of the pressure indicating devices described herein may have utility in the indication of vacuum level in medical devices, including negative pressure wound therapy systems such as the product ranges of Smith & Nephew Medical Ltd (Pico and Renasys), Molnlycke Health Care AB (Avance), ConvaTec Inc. (Avelle) and Kinetic Concepts Inc. (Prevena, Via and Nanova). The pressure indicator can be provided as a self-contained patch that is inserted within a vacuum enclosure of a medical device prior to the commencement of therapy. In pre-constructed wound interfaces, such as Prevena Incision Dressing and Pico dressing, the patch can be integrated in the product construct during manufacture. In use, the patch provides a visual indicator to the carer and user that the system is functioning by providing an indication of pressure applied by the medical device; this may be difficult to achieve by other means in a reliable manner.

Certain embodiments of the pressure indicating devices described herein may have utility in the indication of pressure level in compression garments, medical devices including compression bandaging and/or compressive clothing garments. Compression bandaging can be applied in a manner that generates over- or under-pressure that can be detrimental to the patient. In certain embodiments of the present invention, a pressure indicating device is connected to a flat bladder that can be inserted under the compressive garment at the time of application, see Figure 9. When the appropriate pressure is obtained at the time of device application, the device indicates this accordingly. The indicating device may be left in place or may be removed following device application.

For reversible pressure indication, it is important that the elastomer is non-adhesive to the translucent sheet, and this is true for the non-adhesive elastomers described herein. It will be understood by those skilled in the art that it is also possible to generate irreversible indicator systems based on the general principle described herein. For irreversible indication of pressure, adhesive elastomers can be employed.

Certain embodiments of the pressure indicator devices described herein rely upon a light source for the indication of a pressure and/or change in pressure. This is not a limitation because a source of illumination is required for human vision. In colour indicating devices, a coloured (or non-coloured) reference material is commonly supplied with the indicator. For certain embodiments of the indicators described herein, at least a portion of the coloured elastomer can be deliberately left uncovered by the translucent sheet, providing a reference colour indicating pressurisation. For convenience, the translucent sheet may be designed to be smaller in perimeter than the elastomer, exposing a border of elastomer. Alternatively, the translucent sheet may have an identical perimeter to the elastomer but may have a void, for example, in its centre, through which the elastomer can be viewed. Alternatively, a reference material of the same colour as the elastomer may be provided proximate to the device or separate from it, for example on a reference card.

Certain embodiments of the pressure indicators described herein are capable of both pressure indication and pressure imaging. This is not restricted to a specific pressure range or threshold and the current inventor has exemplified simple single aperture indicators that indicate different pressures in an intuitive manner, based on the size of the circular coloured image formed under pressure. In certain embodiments, the intensity and/or absorbance of the visible colour may be detected.

Certain embodiments of the present invention disclosed herein are distinct from the prior art in the use of a combination of a coloured elastomer with a translucent sheet via an apertured layer. When the sufficiently soft coloured elastomers of certain embodiments and translucent sheet are brought together in to direct contact, the coloured elastomer becomes substantially immediately visible. Without being bound by theory, this is because the suitable combination of materials are able to form a sufficiently intimate contact. The present inventor, without being bound by theory has found that this contact is non-trivial to achieve because the coloured elastomer must conform to the surface texture of the translucent sheet (e.g. structural features and/or the lenticular array) in the absence of an applied pressure (the contact can be formed essentially at no applied pressure if the objects are brought together sufficiently carefully). To achieve suitable conformability, certain embodiments of the present invention employ substantially soft elastomeric materials that are commonly formed by the gellification of liquids.

As used herein the term "soft elastomers" refers to elastomers that have a hardness on the Shore OOO, Shore OO and/or the Shore A hardness scales.

Attention is drawn to "Magic Slate" prior art (refer to Background section) wherein a definite localised pressure is required to generate intimate contact between translucent sheet and waxy substrate. Thus, the prior art and certain embodiments of the present invention disclosed herein function in distinct and mutually exclusive manners: in "Magic Slate" devices, the translucent sheet and waxy layer are free to contact one another prior to use but no image is formed in this state; in certain embodiments of the present invention, direct contact of the translucent sheet and elastomer results in substantially instant visibility of the coloured elastomer. Thus, in the operation of certain embodiments of the present invention, an apertured layer is disposed between the translucent layer and elastomeric layer (e.g. the elastomer of the coloured layer) to help prevent direct contact between these layers prior to or following the application of a sufficient pressure to bring the translucent and elastomeric layers into direct contact via the aperture or apertures of the apertured layer. When the pressure is released, the translucent and elastomeric layers become separated as the elastomer relaxes to its flat initial state.

### Examples

### Example 1 Construction of a pressure indicator with a woven mesh multi-apertured layer, a coloured elastomer and a lenticular lens array

### Formulation of coloured elastomer

100 mg of Solvent Blue 35 (FastColours LLP) was added to 500 g Versagel R1600 (Azelis UK Ltd) in a liquid state (>80 °C). When full dissolution was achieved, the liquid was coated onto 36 micron thickness PET film (PSG Group Ltd) using a slit gap coating machine at a depth of 30 microns. When the film was cool, circles of 25 mm diameter were punched from it using a hydraulic press.

### Construction of pressure indicator

20 mm diameter circles were punched from a hexagonal lenticular lens array sheet (OK3D Technology Co., Ltd) by hydraulic press.

50 mm diameter circles of a monofilament woven mesh with 36 apertures per square centimetre and an open area of 85% (Harrington Fabric and Lace (NLT Integral Ltd), Nottingham UK) were punched by hydraulic press.

A circle of woven mesh was placed directly over a circle of elastomer, elastomer side up. A circle of lens array was placed, lenticular face down, onto the woven mesh layer. All circular layers were centred.

The construct was sealed between layers of perforated transparent adhesive polyurethane film.

### Example 2 Construction of a pressure indicator with a single aperture, a coloured elastomer and a lenticular lens array

### Formulation of coloured elastomer

100 mg of Solvent Blue 35 (FastColours LLP) was added to 500 g Versagel R1600 (Azelis UK Ltd) in a liquid state (>80 °C). When full dissolution was achieved, the liquid was coated onto 36 micron thickness PET film (PSG Group Ltd) using a slit gap coating machine at a depth of 30 microns. When the film was cool, circles of 25 mm diameter were punched from it using a hydraulic press.

### Construction of pressure indicator

25 mm diameter circles were punched from a hexagonal lenticular lens array sheet (OK3D Technology Co., Ltd) by hydraulic press.

Circular annulus of 25 mm outside diameter and 20 mm inside diameter were punched from a polypropylene sheet of 200 micron thickness using a hydraulic press.

A polypropylene annulus was placed directly over a circle of elastomer, elastomer side up. A circle of lens array was placed, lenticular face down, onto the annulus and fixed using adhesive. All circular layers were centred.

### Example 3 Pressure response testing of a pressure indicator (multiple-aperture)

The pressure indicator constructed in Example 1 was inserted under the top film of a 10x20 cm Pico Softport dressing, repairing the top film afterwards with a strip of OpSite Film (Smith & Nephew Medical Ltd). The dressing was evacuated, using a hand-pump, to vacuum pressures in the range of 0 to -120 mmHg relative to atmospheric pressure (745 mmHg) in 20 mmHg increments. Images were recorded of the pressure indicator at each pressure increment and are shown in Figure 10. The resulting images demonstrate that the indicator responds to the internal positive mechanical pressure within the device construct and varies in colour in a predictable manner across the pressure range.

### Example 4 Pressure response testing of a pressure indicator (single aperture)

The pressure indicator constructed in Example 2 was inserted under the top film of a 10x20 cm Pico Softport dressing, repairing the top film afterwards with a strip of OpSite Film (Smith & Nephew Medical Ltd). The dressing was evacuated, using a hand-pump, to vacuum pressures in the range of 0 to -70 mmHg relative to atmospheric pressure (745 mmHg) in 10 mmHg increments. Images were recorded of the pressure indicator at each pressure increment and are shown in Figure 11. The resulting images demonstrate that the indicator responds to the internal mechanical pressure within the device construct and coloured circles of varying diameter are produced in a predictable manner across the pressure range.

### Example 5 Construction of a pressure indicator with a woven mesh multi-apertured layer, a coloured elastomer and a translucent sheet (PVC)

### Formulation of coloured elastomer

100 mg of Solvent Blue 35 (FastColours LLP) was added to 500 g Versagel R1600 (Azelis UK Ltd) in a liquid state (>80 °C). When full dissolution was achieved, the liquid was coated onto 36 micron thickness PET film (PSG Group Ltd) using a slit gap coating machine at a depth of 30 microns. When the film was cool, circles of 25 mm diameter were punched from it using a hydraulic press.

### Construction of pressure indicator

25 mm diameter circles were punched from a sheet of opacified PVC of 100 um thickness. Circular annulus of 25 mm outside diameter and 20 mm inside diameter were punched from a polypropylene sheet of 200 micron thickness using a hydraulic press.

A polypropylene annulus was placed directly over a circle of elastomer, elastomer side up. The circle of opacified PVC was placed onto the annulus and fixed using adhesive. All circular layers were centred.

### Example 6 Construction of a pressure indicator with a woven mesh multi-apertured layer, a coloured elastomer and a translucent sheet (PET)

### Formulation of coloured elastomer

100 mg of Solvent Blue 35 (FastColours LLP) was added to 500 g Versagel R1600 (Azelis UK Ltd) in a liquid state (>80 °C). When full dissolution was achieved, the liquid was coated onto 36 micron thickness PET film (PSG Group Ltd) using a slit gap coating machine at a depth of 30 microns. When the film was cool, circles of 25 mm diameter were punched from it using a hydraulic press.

### Construction of pressure indicator

25 mm diameter circles were punched from a sheet of opacified PET (Mylar A) of 75 um thickness.

Circular annulus of 25 mm outside diameter and 20 mm inside diameter were punched from a polypropylene sheet of 200 micron thickness using a hydraulic press.

A polypropylene annulus was placed directly over a circle of elastomer, elastomer side up. The circle of opacified PET was placed onto the annulus and fixed using adhesive. All circular layers were centred.

### Example 7 Construction of a pressure indicator with a single aperture, a coloured elastomer and a lenticular lens array

### Formulation of coloured elastomer

100 mg of Solvent Red 35 (FastColours LLP) was added to 500 g Versagel R1600 (Azelis UK Ltd) in a liquid state (>80 °C). When full dissolution was achieved, the liquid was coated onto 36 micron thickness PET film (PSG Group Ltd) using a slit gap coating machine at a depth of 30 microns. When the film was cool, circles of 25 mm diameter were punched from it using a hydraulic press.

### Construction of pressure indicator

25 mm diameter circles were punched from a hexagonal lenticular lens array sheet (OK3D Technology Co., Ltd) by hydraulic press.

Circular annulus of 25 mm outside diameter and 20 mm inside diameter were punched from a polypropylene sheet of 200 micron thickness using a hydraulic press.

A polypropylene annulus was placed directly over a circle of elastomer, elastomer side up. A circle of lens array was placed, lenticular face down, onto the annulus and fixed using adhesive. All circular layers were centred.

### Example 8 Construction of a pressure indicator with a single aperture, a coloured elastomer and a lenticular lens array (cellulose acetate)

### Formulation of coloured elastomer

100 mg of Solvent Red 35 (FastColours LLP) was added to 500 g Versagel R1600 (Azelis UK Ltd) in a liquid state (>80 °C). When full dissolution was achieved, the liquid was coated onto 36 micron thickness PET film (PSG Group Ltd) using a slit gap coating machine at a depth of 30 microns. When the film was cool, circles of 25 mm diameter were punched from it using a hydraulic press.

### Construction of pressure indicator

25 mm diameter circles were punched from a sheet of opacified cellulose acetate sheet by hydraulic press.

Circular annulus of 25 mm outside diameter and 20 mm inside diameter were punched from a polypropylene sheet of 200 micron thickness using a hydraulic press.

A polypropylene annulus was placed directly over a circle of elastomer, elastomer side up. A circle of opacified cellulose acetate film was placed onto the annulus and fixed using adhesive. All circular layers were centred.

### Example 9 Construction of a pressure indicator with a woven mesh multi-apertured layer, a coloured elastomer and a lenticular lens array (silicone elastomer)

### Formulation of coloured elastomer

1 g of Blue Elastosil AUX Color Paste FL (Wacker Chemie AG) was added to 100 g Silpuran 2420/30 part B (Wacker Chemie AG) and mixed thoroughly to achieve colour uniformity. This was thoroughly mixed with 100 g Silpuran 2420/30 part A (Wacker Chemie AG) and the resulting homogeneous mixture coated onto 36 micron thickness PET film (PSG Group Ltd) using a slit gap coating machine at a depth of 30 microns. The so-formed film was cured under infra-red lamps at 80 °C for 15 minutes. When the film was cool, circles of 25 mm diameter were punched from it using a hydraulic press.

### Construction of pressure indicator

20 mm diameter circles were punched from a hexagonal lenticular lens array sheet (OK3D Technology Co., Ltd) by hydraulic press.

50 mm diameter circles of a monofilament woven mesh with 36 apertures per square centimetre and an open area of 85% (Harrington Fabric and Lace (NLT Integral Ltd), Nottingham UK) were punched by hydraulic press.

A circle of woven mesh was placed directly over a circle of elastomer, elastomer side up. A circle of lens array was placed, lenticular face down, onto the woven mesh layer. All circular layers were centred.

The construct was sealed between layers of perforated transparent adhesive polyurethane film.

### Example 10 Construction of a pressure indicator with a woven mesh multi-apertured layer, a coloured elastomer and a lenticular lens array (silicone elastomer)

### Formulation of coloured elastomer

1 g of Blue Elastosil AUX Color Paste FL (Wacker Chemie AG) was added to 100 g Elastosil P7600 part B (Wacker Chemie AG) and mixed thoroughly to achieve colour uniformity. This was thoroughly mixed with 100 g Elastosil P7600 part A (Wacker Chemie AG) and the resulting homogeneous mixture coated onto 36 micron thickness PET film (PSG Group Ltd) using a slit gap coating machine at a depth of 30 microns. The so-formed film was cured under infra-red lamps at 80 °C for 15 minutes. When the film was cool, circles of 25 mm diameter were punched from it using a hydraulic press.

### Construction of pressure indicator

20 mm diameter circles were punched from a hexagonal lenticular lens array sheet (OK3D Technology Co., Ltd) by hydraulic press.

50 mm diameter circles of a monofilament woven mesh with 36 apertures per square centimetre and an open area of 85% (Harrington Fabric and Lace (NLT Integral Ltd), Nottingham UK) were punched by hydraulic press.

A circle of woven mesh was placed directly over a circle of elastomer, elastomer side up. A circle of lens array was placed, lenticular face down, onto the woven mesh layer. All circular layers were centred.

The construct was sealed between layers of perforated transparent adhesive polyurethane film.

### Example 11 Construction of a pressure indicator with a woven mesh multi-apertured layer, a coloured elastomer and a lenticular lens array (silicone elastomer)

### Formulation of coloured elastomer

1 g of Blue Elastosil AUX Color Paste FL (Wacker Chemie AG) was added to 100 g Elastosil P7676 part B (Wacker Chemie AG) and mixed thoroughly to achieve colour uniformity. This was thoroughly mixed with 100 g Elastosil P7676 part A (Wacker Chemie AG) and the resulting homogeneous mixture coated onto 36 micron thickness PET film (PSG Group Ltd) using a slit gap coating machine at a depth of 30 microns. The so-formed film was cured under infra-red lamps at 80 °C for 15 minutes. When the film was cool, circles of 25 mm diameter were punched from it using a hydraulic press.

### Construction of pressure indicator

20 mm diameter circles were punched from a hexagonal lenticular lens array sheet (OK3D Technology Co., Ltd) by hydraulic press.

50 mm diameter circles of a monofilament woven mesh with 36 apertures per square centimetre and an open area of 85% (Harrington Fabric and Lace (NLT Integral Ltd), Nottingham UK) were punched by hydraulic press.

A circle of woven mesh was placed directly over a circle of elastomer, elastomer side up. A circle of lens array was placed, lenticular face down, onto the woven mesh layer. All circular layers were centred.

The construct was sealed between layers of perforated transparent adhesive polyurethane film.

## Claims

1. A device for reversible indication of applied pressure by a colour change comprising;
a translucent layer;
a coloured layer comprising an elastomer; and
an apertured layer, comprising one or more apertures;
wherein the apertured layer is disposed between the translucent layer and the coloured layer.

2. A device according to claim 1, wherein the translucent layer comprises at least one textured surface.

3. A device according to claim 2, wherein the at least one textured surface comprises one or more regular or irregular structural features comprising one or more projections with at least one dimension in the range of 100 nanometres to 1 millimetre.

4. A device according to any of claims 2 to 3, wherein the at least one textured surface comprises a continuous layer comprising air filled voids, optionally wherein the translucent layer comprises an opacified polymer and further optionally wherein the opacified polymer is chosen from: poly(ethylene terephthalate), poly(vinyl chloride) or cellulose acetate.

5. A device according to any of claims 1 to 2, wherein the translucent layer comprises a lenticular lens array sheet having a tetragonal or hexagonal close-packed lens packing arrangement and has a lens packing density within a range of 50-500 lenses per linear inch.

6. A device according to claim 5, wherein the lenticular lens array sheet comprises a first surface and a second surface opposed to the first surface wherein the lenticular lenses are disposed on the first surface and the second surface is flat.

7. A device according to any preceding claim, wherein the translucent layer is configured to return 50-70% of an intensity of light incident to a face of the translucent layer to the face of the translucent layer to which the light was incident.

8. A device according to any preceding claim, wherein the coloured layer comprises a coloured elastomer, and optionally wherein the coloured elastomer comprises a solvent dye, and further optionally wherein the coloured layer comprises a transparent elastomer associated with a coloured backing layer.

9. A device according to claim 8, wherein the elastomer is selected from a hydrocarbon-based gel, a polyurethane gel, a hydrogel and a silicone based gel and optionally wherein the elastomer has a Shore hardness on the Shore OOO, Shore OO or Shore A scales.

10. A device according to any preceding claim, wherein each of the elastomer and the translucent layer have a refractive index in the range of 1.30-1.71.

11. A device according to any preceding claim, wherein the apertured layer comprises a single aperture of regular geometry, wherein optionally the single aperture comprises a circular geometry of diameter within a range of 0.1-50 mm.

12. A device according to any of claims 1 to 10, wherein the apertured layer comprises multiple identical apertures of regular geometry, wherein optionally each of the apertures has a hexagonal, tetragonal or square geometry with a major axis length within a range of 0.01-5 mm.

13. A device according to any preceding claim for the indication of a pressure above or below ambient atmospheric pressure.

14. A device according to any preceding claim for the indication of a pressure level within a medical device, including positive and negative pressures, including: negative pressure wound therapy devices and compression garments including compression bandaging

15. A clothing garment comprising a device according to any one of claims 1 to 14.

## Patentansprüche

1. Vorrichtung zur umkehrbaren Anzeige eines angewandten Drucks durch eine Farbänderung, umfassend:
eine lichtdurchlässige Schicht;
eine farbige Schicht, umfassend ein Elastomer; und
eine mit Öffnungen versehene Schicht, umfassend eine oder mehrere Öffnungen;
wobei die mit Öffnungen versehene Schicht zwischen der lichtdurchlässigen Schicht und der farbigen Schicht angeordnet ist.

2. Vorrichtung nach Anspruch 1, wobei die lichtdurchlässige Schicht mindestens eine texturierte Oberfläche umfasst.

3. Vorrichtung nach Anspruch 2, wobei die mindestens eine texturierte Oberfläche eine oder mehrere regelmäßige oder unregelmäßige strukturelle Merkmale umfasst, umfassend einen oder mehrere Vorsprünge mit mindestens einer Abmessung im Bereich von 100 Nanometern bis 1 Millimeter.

4. Vorrichtung nach einem der Ansprüche 2 bis 3, wobei die mindestens eine texturierte Oberfläche eine durchgängige Schicht, umfassend luftgefüllte Hohlräume, umfasst, optional wobei die lichtdurchlässige Schicht ein getrübtes Polymer umfasst und ferner optional wobei das getrübte Polymer ausgewählt ist aus: Poly(ethylenterephtalat), Poly(vinylchlorid) oder Zelluloseacetat.

5. Vorrichtung nach einem der Ansprüche 1 bis 2, wobei die lichtdurchlässige Schicht eine linsenförmige Linsenanordnungslage mit einer tetragonalen oder hexagonalen, eng gepackten Linsenpackanordnung aufweist, und eine Linsenpackdichte in einem Bereich von 50 bis 500 Linsen je linearem Zoll aufweist.

6. Vorrichtung nach Anspruch 5, wobei die linsenförmige Linsenanordnungslage eine erste Oberfläche und eine der ersten Oberfläche gegenüberliegende zweite Oberfläche umfasst, wobei die linsenförmigen Linsen auf der ersten Oberfläche angeordnet sind und die zweite Oberfläche flach ist.

7. Vorrichtung nach einem der vorangehenden Ansprüche, wobei die lichtdurchlässige Schicht konfiguriert ist, um 50 bis 70 % einer Intensität von auf eine Seite der lichtdurchlässigen Schicht auftreffendes Licht zu der Seite der lichtdurchlässigen Schicht, auf die das Licht aufgetroffen ist, zurückzugeben.

8. Vorrichtung nach einem der vorangehenden Ansprüche, wobei die farbige Schicht ein farbiges Elastomer umfasst und optional wobei das farbige Elastomer einen Lösungsmittelfarbstoff umfasst, und ferner optional wobei die farbige Schicht ein transparentes Elastomer umfasst, das einer farbigen Trägerschicht zugeordnet ist.

9. Vorrichtung nach Anspruch 8, wobei das Elastomer ausgewählt ist aus einem kohlenwasserstoffbasierten Gel, einem Polyurethangel, einem Hydrogel und einem siliziumbasierten Gel, und optional wobei das Elastomer eine Shore-Härte auf der Shore OOO-, Shore OO- oder Shore A-Skala aufweist.

10. Vorrichtung nach einem der vorangehenden Ansprüche, wobei jede der Elastomer- und der lichtdurchlässigen Schicht einen Brechungsindex im Bereich von 1,30 bis 1,71 aufweist.

11. Vorrichtung nach einem der vorangehenden Ansprüche, wobei die mit Öffnungen versehene Schicht eine einzelne Öffnung mit regelmäßiger Geometrie umfasst, wobei optional die einzige Öffnung eine kreisförmige Geometrie eines Durchmessers innerhalb eines Bereichs von 0,1 bis 50 mm aufweist.

12. Vorrichtung nach einem Ansprüche 1 bis 10, wobei die mit Öffnungen versehene Schicht mehrere identische Öffnungen mit regelmäßiger Geometrie umfasst, wobei optional jede der Öffnungen eine hexagonale, tetragonale oder quadratische Geometrie mit einer Hauptachslänge innerhalb eines Bereichs von 0,01 bis 5 mm aufweist.

13. Vorrichtung nach einem der vorangehenden Ansprüche zur Anzeige eines Drucks über oder unter Umgebungsdruck.

14. Vorrichtung nach einem der vorangehenden Ansprüche zur Anzeige eines Druckniveaus in einer medizinischen Vorrichtung, einschließlich positiver und negativer Drucke, enthaltend: Negativdruck-Wundtherapievorrichtungen und Kompressionskleidungsstücke, einschließlich Kompressionsverbänden.

15. Bekleidungsstück, umfassend eine Vorrichtung nach einem der Ansprüche 1 bis 14.

## Revendications

1. Dispositif d'indication réversible de pression appliquée par un changement de couleur, comprenant :
une couche translucide ;
une couche colorée comprenant un élastomère ; et
une couche perforée, comprenant une ou plusieurs perforations ; dans lequel la couche perforée est disposée entre la couche translucide et la couche colorée.

2. Dispositif selon la revendication 1, dans lequel la couche translucide comprend au moins une surface texturée.

3. Dispositif selon la revendication 2, dans lequel l'au moins une surface texturée comprend une ou plusieurs caractéristiques structurelles régulières ou irrégulières comprenant une ou plusieurs saillies avec au moins une dimension dans la plage de 100 nanomètres à 1 millimètre.

4. Dispositif selon l'une quelconque des revendications 2 à 3, dans lequel l'au moins une surface texturée comprend une couche continue comprenant des vides remplis d'air, facultativement dans lequel la couche translucide comprend un polymère opacifié et en outre facultativement dans lequel le polymère opacifié est choisi parmi : le poly (éthylène téréphtalate), poly (chlorure de vinyle) ou acétate de cellulose.

5. Dispositif selon l'une quelconque des revendications 1 à 2, dans lequel la couche translucide comprend une feuille à réseau de lentilles lenticulaires ayant un agencement à assemblage tétragonal ou hexagonal compact de lentilles et a une densité d'assemblage de lentilles dans une plage de 50 à 500 lentilles par pouce linéaire.

6. Dispositif selon la revendication 5, dans lequel la feuille de réseau de lentilles lenticulaires comprend une première surface et une seconde surface opposée à la première surface dans lequel les lentilles lenticulaires sont disposées sur la première surface et la seconde surface est plate.

7. Dispositif selon l'une quelconque revendication précédente, dans lequel la couche translucide est configurée pour renvoyer 50 à 70% d'une intensité de lumière incidente à une face de la couche translucide à la face de la couche translucide à laquelle la lumière était incidente.

8. Dispositif selon l'une quelconque revendication précédente, dans lequel la couche colorée comprend un élastomère coloré, et éventuellement dans lequel l'élastomère coloré comprend un colorant solvant, et en outre facultativement dans lequel la couche colorée comprend un élastomère transparent associé à une couche de support colorée.

9. Dispositif selon la revendication 8, dans lequel l'élastomère est choisi parmi un gel à base d'hydrocarbure, un gel de polyuréthane, un hydrogel et un gel à base de silicone et facultativement dans lequel l'élastomère a une dureté Shore sur les échelles Shore OOO, Shore OO ou Shore A.

10. Dispositif selon l'une quelconque revendication précédente, dans lequel chacun de l'élastomère et de la couche translucide a un indice de réfraction dans la plage de 1,30 à 1,71.

11. Dispositif selon l'une quelconque revendication précédente, dans lequel la couche perforée comprend une seule perforation de géométrie régulière, dans lequel facultativement la seule perforation comprend une géométrie circulaire de diamètre dans une plage de 0,1 à 50 mm.

12. Dispositif selon l'une quelconque des revendications 1 à 10, dans lequel la couche perforée comprend plusieurs perforations identiques de géométrie régulière, dans lequel facultativement chacune des perforations a une géométrie hexagonale, tétragonale ou carrée avec une longueur d'axe principal dans une plage de 0,01 à 5 mm.

13. Dispositif selon l'une quelconque revendication précédente permettant l'indication d'une pression supérieure ou inférieure à la pression atmosphérique ambiante.

14. Dispositif selon l'une quelconque revendication précédente permettant l'indication d'un niveau de pression à l'intérieur d'un dispositif médical, comprenant des pressions positives et négatives, comprenant : des dispositifs de traitement des plaies à pression négative et des vêtements de compression comprenant un bandage de compression.

15. Vêtement vestimentaire comprenant un dispositif selon l'une quelconque des revendications 1 à 14.
